# EUROPEAN PATENT SPECIFICATION

(11) **EP 4 561 647 B1**
(45) Date of publication and mention of the grant of the patent: **04.11.2026**
(21) Application number: 23749060.2
(22) Date of filing: 28.07.2023
(51) Int. Cl.: A61L 27/26, A61L 27/52, A61L 27/54

(54) **HYDROGEL COMPOSITION FOR USE IN THE TREATMENT OF ARTICULAR DISORDERS**
HYDROGELZUSAMMENSETZUNG ZUR VERWENDUNG BEI DER BEHANDLUNG VON GELENKSTÖRUNGEN
COMPOSITION D'HYDROGEL POUR UNE UTILISATION DANS LE TRAITEMENT DE TROUBLES ARTICULAIRES

(30) Priority: 28.07.2022 EP 22306135
(43) Date of publication of application: 04.06.2025
(73) Proprietor: Université de Lille, 59800 Lille (FR); Institut National de la Santé et de la Recherche Médicale, 75013 Paris (FR); Centre Hospitalier Universitaire de Lille, 59000 Lille (FR); Institut National de Recherche pour l'Agriculture, l'Alimentation et l'Environnement, 75007 Paris (FR); Centre National de la Recherche Scientifique (CNRS), 75016 Paris (FR); Centrale Lille Institut, 59650 Villeneuve-d'Ascq (FR)
(72) Inventor: BLANCHEMAIN, Nicolas, 59660 MERVILLE (FR); CHAI, Feng, 59120 LOOS (FR); NICOT, Romain, 59800 LILLE (FR); MARTEL, Bernard, 59850 NIEPPE (FR); GARCIA FERNANDEZ, Maria José, 59000 LILLE (FR); TABARY, Nicolas, 59000 LILLE (FR); CHIJCHEAPAZA-FLORES, Henry, 59800 LILLE (FR)
(74) Representative: Regimbeau
(86) International application number: PCT/EP2023/071092
(87) International publication number: WO 2024/023350

(56) References cited:
- WO-A1-2017/001808
- US-A1- 2018 000 737
- US-A1- 2021 100 832
- PATCHORNIK SHACHAR ET AL: "Chitosan-Hyaluronate Hybrid Gel Intraarticular Injection Delays Osteoarthritis Progression and Reduces Pain in a Rat Meniscectomy Model as Compared to Saline and Hyaluronate Treatment", vol. 2012, 1 January 2012 (2012-01-01), pages 1 - 5, XP093007564, ISSN: 2090-3464, Retrieved from the Internet <URL:https://downloads.hindawi.com/journals/aorth/2012/979152.pdf> DOI: 10.1155/2012/979152

## Description

### FIELD OF THE INVENTION

The present invention relates to a hydrogel composition based on anionic cyclodextrin polymer and chitosan for use in the treatment of articular disorders. In particular it relates to a hydrogel composition based on cyclodextrin polymers and chitosan for use in the treatment of articular disorders and which combines a pharmacological action, in particular an analgesic action, with a visco-supplementation, i.e. a lubricating effect.

### BACKGROUND OF THE INVENTION

Temporomandibular disorders (TMDs) also known as temporomandibular joint dysfunctions (TMJD) encompass disorders that affect the temporomandibular joints (the joints that connect the mandible to the skull). TMDs are myo-arthropathies of the manducatory apparatus which are responsible for chronic pain symptoms and restricted jaw movements. TMDs are the first cause of orofacial pain in the world and would affect 5 to 12% of the population in industrialized countries. According to the American Academy of Orofacial Pain, TDMs encompass a group of musculoskeletal and neuromuscular pathologies that involve the temporomandibular joints, masticatory muscles, and all associated tissues. Among the pain symptoms, those whose manifestation is more muscular (temporal muscles and / or masseter muscles) can be differentiated from those whose manifestation is rather articular. In this case, there is often an anatomical substratum responsible for the pain (e.g. disc displacement which may or may not be reducible, degenerative joint disease including osteoarthrosis and osteoarthritis). Aetiologies are numerous and are frequently associated: excessive mechanical stress, trauma of the condylar area, dental malocclusion, systemic illness, hormonal and genetic factors (Tanaka E, Detamore MS, Mercuri LG. Degenerative disorders of the temporomandibular joint: etiology, diagnosis, and treatment. J Dent Res. avr 2008;87(4):296-307. Wang XD, Zhang JN, Gan YH, Zhou YH. Current understanding of pathogenesis and treatment of TMJ osteoarthritis. J Dent Res. mai 2015;94(5):666-73). All these causes lead to a dysfunctional remodelling of the joint components. Indeed, hypoxia induced by such factors, result in an up-regulation of vascular endothelial growth factor (VEGF) production (Tanaka E, Detamore MS, Mercuri LG. Degenerative disorders of the temporomandibular joint: etiology, diagnosis, and treatment. J Dent Res. avr 2008;87(4):296-307). VEGF increases the production of matrix metalloproteinases (MMPs) and reduces that of tissue-inhibitor of matrix metalloproteinases (TIMPs). This imbalance of effectors of extracellular matrix remodelling accelerates bone and cartilage resorption. Hypoxia induced by overloading also causes release of free oxygen radicals and pro-inflammatory cytokines such as interleukin-1β (IL-1β); interleukin-6 (IL-6) and tumour necrosis factor-α (TNFα). These cytokines regulate the production of hyaluronic acid (HA) and in such case of high level of pro-inflammatory cytokines, HA is degraded leading to a collapse of joint lubrication. The loss of synovial fluid and so the loss of viscosity impair nutritional support to the cartilage result in its progressive degradation (Ernberg M. The role of molecular pain biomarkers in temporomandibular joint internal derangement. J Oral Rehabil. juin 2017;44(6):481-91). In the same way, IL-1β and TNF-α promote the production of MMP and collagenase by the synovial cells and articular chondrocytes (Tabeian H, Bakker AD, Betti BF, Lobbezoo F, Everts V, de Vries TJ. Cyclic Tensile Strain Reduces TNF-α Induced Expression of MMP-13 by Condylar Temporomandibular Joint Cells. J Cell Physiol. juin 2017;232(6):1287-94. Hutchinson NI, Lark MW, MacNaul KL, Harper C, Hoerrner LA, McDonnell J, et al. In vivo expression of stromelysin in synovium and cartilage of rabbits injected intraarticularly with interleukin-1 beta. Arthritis Rheum. oct 1992;35(10):1227-33. Kubota E, Imamura H, Kubota T, Shibata T, Murakami K. Interleukin 1 beta and stromelysin (MMP3) activity of synovial fluid as possible markers of osteoarthritis in the temporomandibular joint. J Oral Maxillofac Surg. janv 1997;55(1):20-7; discussion 27-28) as well as the activation of cyclooxygenase 1 and 2 (COX-1/2) which enhance inflammation by increasing prostaglandin E2 (PGE2) level (Satoh K, Ogura N, Akutsu M, Kuboyama N, Kuyama K, Yamamoto H, et al. Expression of cyclooxygenase-1 and -2 in IL-1beta-induced synovitis of the temporomandibular joint. J Oral Pathol Med. août 2009;38(7):584-90. Emshoff R, Puffer P, Rudisch A, Gassner R. Temporomandibular joint pain: relationship to internal derangement type, osteoarthrosis, and synovial fluid mediator level of tumor necrosis factor-alpha. Oral Surg Oral Med Oral Pathol Oral Radiol Endod. oct 2000;90(4):442-9). All these pathways play a role in TMJ osteoarthritis pathogenesis by accelerating the cartilage and bone degradation, reducing synovial fluid amount and creating chronic inflammation.

Symptoms are related to persistent inflammatory process and osteoarthrosis composing the osteoarthritis. The main symptom is chronic orofacial pain, it the result of chronic synovitis, reactional muscular spasms and degenerative arthropathy (Ahmad M, Schiffman EL. Temporomandibular Joint Disorders and Orofacial Pain. Dent Clin North Am. janv 2016;60(1):105-24). Other symptoms are usually founded in case of TMJOA: jaw functional limitation: reduced jaw mobility, verbal expression and chewing limitation, mouth opening limitation, but also palpation and neck pain, joint noises (Meloto CB, Slade GD, Lichtenwalter RN, Bair E, Rathnayaka N, Diatchenko L, et al. Clinical predictors of persistent temporomandibular disorder in people with first-onset temporomandibular disorder: A prospective case-control study. J Am Dent Assoc. juill 2019;150(7):572-581.e10. Ohrbach R, Fillingim RB, Mulkey F, Gonzalez Y, Gordon S, Gremillion H, et al. Clinical findings and pain symptoms as potential risk factors for chronic TMD: descriptive data and empirically identified domains from the OPPERA case-control study. J Pain. nov 2011;12(11 Suppl):T27-45). Symptoms have a variable intensity through time, alternating phases of exacerbation but can also become chronic.

Many therapeutic approaches have been considered in the complex management of these TMDs. Classical non-invasive therapies combine physiotherapy sessions, occlusal treatments (often requiring an occlusal release splint) and symptomatic treatments administered systemically which combine analgesics and muscle relaxants. Nonsteroidal anti-inflammatory drugs (NSAIDs) are commonly prescribed by oral route to reduce chronic inflammation but they are related to negative side effects due to a systemic action. For articular forms, in case occlusal treatment has failed, treatment modalities are often mini-invasive, involving joint lavage (arthrocentesis) and / or intra-articular injection of various substances.

Hyaluronic acid with its well-known lubricating properties, as well as corticoids, morphine and its derivatives, ketamine or botulinum toxin, with analgesic properties, or even platelet-rich plasma (PRP) which helps regeneration, are among the pharmacological substances used in intra-articular injections with variable results.

Intra-articular injection of pharmacological substances avoids their systemic effects (gastric and renal tolerance of non-steroidal anti-inflammatory drugs, metabolic problems for corticosteroids) (Al-Moraissi EA et al., J Cranio-Maxillo-fac Surg 2020;48(1):9-23). Supports based on biomaterials (nanoparticles, microparticles or hydrogel) have been proposed in order to administer pharmacological substances in a controllable manner at injected anatomical site (Systematic review of studies on drug-delivery systems for management of temporomandibular-joint osteoarthritis. Barry F, Chai F, Chijcheapaza-Flores H, Garcia-Fernandez MJ, Blanchemain N, Nicot R. J Stomatol Oral Maxillofac Surg. 2021 Aug 13:S2468-7855(21)00163-4. doi: 10.1016/j.jormas.2021.08.003. Online ahead of print). However, as stated in the state of the art, the release must remain within the effective therapeutic window: the amount of active ingredient released must be sufficient to be effective in the long term but not too important to prevent local cytotoxicity in the joint and damaging the surrounding tissue (cartilage) (Cicognani M. et al., Pharmaceutics 2020;12(7):681. Deng Y et al., Sci Rep 2017; Kou L et Al., Drug Deliv 2019;26(1):870-85). US 2021/100832 discloses compositions for use in treating musculoskeletal disorder *e.g*. arthritis, osteoarthritis, comprising therapeutically effective amounts of a galectin inhibitor comprising galactoarabino-rhamnogalacturonan and/or galactomannan, administered in combination with hyaluronic acid and chitosan.

Currently, there is no available injectable substance that combines the advantages of viscosupplementation (as offered by hyaluronic acid) with an efficient and safe pharmacological action. The pharmacological substances currently used suffer from a lack of prolonged effects (for non-steroidal anti-inflammatory drugs) or from harmful local consequences by inducing bone demineralization (for corticosteroids).

Therefore, a need remains for an injectable composition for use in the treatment of TMDs, more broadly for use in the treatment of articular disorders, which combines a relief of pain with a lubricating effect (viscosupplementation). More specifically, a need remains for an injectable composition for use in the treatment of TMDs, more broadly for use in the treatment of articular disorders, which combines a pharmacological action with a lubricating effect (viscosupplementation), when such a dual effect is desired or required.

### SUMMARY OF THE INVENTION

The invention relates to a hydrogel composition comprising:
(a) a hydrogel made of a polymer network comprising:
   (i) chitosan;
   (ii) anionic cyclodextrin polymers,
      said anionic cyclodextrin polymers being water soluble anionic cyclodextrin polymers or a mixture of water soluble anionic cyclodextrin polymers and water insoluble anionic cyclodextrin polymers (CDPi) wherein the ratio of the weight of water soluble anionic cyclodextrin polymers to the weight of water insoluble cyclodextrin polymers is about 1:1;
      wherein the ratio of the weight of the chitosan to the total weight of the anionic cyclodextrin polymers is about 2:1; and
(b) optionally a pharmacological agent incorporated in the polymer network,
for use in the treatment of articular disorders.

The invention also relates to a method for preparing a hydrogel composition comprising a pharmacological agent, said method comprising the following steps:
(i) providing powders of water soluble anionic cyclodextrin polymer, water insoluble anionic cyclodextrin polymer powder when applicable and chitosan;
(ii) adding by wet granulation a pharmacological agent to the water soluble anionic cyclodextrin polymer powder or to the mixture of water soluble and water insoluble anionic cyclodextrin polymer powders;
(iii) dry mixing the powder obtained in step (ii) with chitosan powder in order to form a mixture of powders;
(iv) optionally sieving, or co-milling and sieving, the mixture of powders obtained in step (iii);
(v) suspending the mixture of powders obtained in step (iv) in an aqueous medium having a pH that enables the anionic cyclodextrin polymer(s) to be dissolved without dissolving the chitosan;
(vi) acidifying the suspension obtained from step (v) in order to form the hydrogel composition.

Further aspects of the invention are as disclosed herein and in the claims.

### FIGURES

Figure 1: Reversed-vial test at 37°C for CHT/PCDs/PCDi hydrogels and R.P., evaluation of hydrogel formation by flowing time.
Figure 2: Injection of hydrogel in PBS (80 rpm at 37°C) to study the hydrogel stability and cohesion.
Figure 3: Viscoelastic modulus evaluation in the viscoelastic range of non-sterilized hydrogels and R.P. a) Modulus G' and G" at an amplitude 1% and a frequency of 10 rad/s; b) Damping Factor at an amplitude 1% and a frequency of 10 rad/s.
Figure 4: Viscosity curve of non-sterilized hydrogels and R.P., shear rate from 0 to 1000 s⁻¹.
Figure 5: Evaluation of the recovery of non-sterilized hydrogels and R.P. by the cyclic deformation method at shear amplitudes cycles γ₀=1% during 180 seconds and γ₁=500% during 120 seconds at 25°C.
Figure 6: Viscoelastic modulus evaluation in the viscoelastic range of hydrogels with active molecule (CHT + PCDs/PCDi/NaNX). G' and G" Modulus at an amplitude of 1% and frequency of 10 rad/sec.
Figure 7: Viscosity curve of hydrogels with active molecule (CHT + PCDs/PCDi/NaNX), shear rate sweep from 0 to 1000 s-1.
Figure 8: Evaluation of recovery (CHT + PCDs/PCDi/NaNX) by the cyclic deformation method at shear amplitude cycles γ₀=1% during 180 seconds and γ₁=500% during 120 seconds, 25°C.
Figure 9: Evaluation of drug release in a USP 1 dissolution apparatus at 37°C (PBS pH 7.4).
Figure 10: Fibroblasts cells line viability by the extract method from hydrogel CHT/PCDs/PCDi.
Figure 11: Evaluation of Torque over 4 cycles (30 minutes, 25°C and 45N) by using a PTFE-PTFE pin on disk system.
Figure 12: Sterilization impact on viscoelastic modulus of hydrogels with and without active molecule. G' and G" Modulus at an amplitude of 1% and frequency of 10 rad/sec.
Figure 13: Fibroblasts cells line viability by the extract method from sterilized hydrogel.
Figure 14: Pain experienced by the rat after intra-TMJ injection of chemical agent.

### DESCRIPTION OF THE INVENTION

The present invention relates to a hydrogel composition as disclosed herein for use in the treatment of articular disorders. The hydrogel composition allows providing both a relief of pain and a lubricating effect (viscosupplementation), and this without the need to administrate a pharmacological agent. Thus, in some embodiments, the hydrogel composition is free from any pharmacological agent, in particular free from pharmacological agents as disclosed herein below.

In some embodiments, the local release of a pharmacological agent may be desired or required. The hydrogel composition then comprises a pharmacological agent.

Examples of suitable articular disorders that may be treated thanks to the hydrogel composition of the present invention include, but are not limited to, osteoarthritis, osteoarthrosis (e.g. gonarthrosis), rheumatic disorders (e.g. rheumatoid arthritis or juvenile idiopathic arthritis), degenerative meniscal lesions.

Osteoarthritis is one of the most common joint diseases and can affect all the human (the shoulders, the knees, and the temporomandibular joint). In some embodiments, the hydrogel composition is for use in the treatment of temporomandibular joint disorders.

The pharmacological action may be achieved through the release of a pharmacological agent. Preferably an analgesic action, i.e. a relieve of pain, is achieved through the release of an analgesic agent from the hydrogel. Examples of suitable analgesic agents include, but are not limited to, opioid analgesic agents and nonsteroidal anti-inflammatory analgesic agents. Examples of opioid analgesic agents include codeine, oxycodone and eventually hydromorphone in severe pain. Examples of nonsteroidal anti-inflammatory agents include naproxen, ibuprofen, diflunisal and ketorolac. In the case of rheumatic disorders such as rheumatoid arthritis or juvenile idiopathic arthritis, the composition could include anti-TNFα drugs.

In some embodiments, the invention relates to a hydrogel composition as disclosed herein for use in the treatment of osteoarticular pain, in particular for use in the treatment of the temporomandibular joint pain. In some embodiments, the invention relates to a hydrogel composition for use in postoperative pain control after arthroscopy or open joint surgery.

The composition of the present invention is based on a hydrogel. A hydrogel is made of a polymer network which is expanded by water, in particular a large quantity of water. In a hydrogel, the bonds between the polymer chains may be permanent (covalent bonds) or reversible (non-covalent bonds, e.g. hydrogen bonds, ionic bonds, hydrophobic bonds, or dipole-dipole bonds). Hydrogels with permanent bonds and hydrogels with reversible bonds are respectively known as chemical hydrogels and physical hydrogels. The present invention relates to a composition based on a physical hydrogel crosslinked by ionic bonds between chitosan as positive polyelectrolyte, and cyclodextrin polymers as negative polyelectrolyte. These ionic bonds are weak non covalent interactions that endow visco-elastic, shear thickening and self-healing that result in the injectable properties to these mixtures.

It is understood that the term "cyclodextrin polymer" as used herein refers to anionic cyclodextrin polymer, including when used in the expression "water soluble cyclodextrin polymer" and "water insoluble cyclodextrin polymer".

The hydrogel composition of the present invention has been found to be biocompatible, stable upon and after sterilization and exhibiting properties making it suitable for administration by injection, for instance by means of a syringe. The hydrogel composition of the present invention exhibits suitable viscoelastic properties (Shear-thinning and self-healing properties). The hydrogel composition fluidifies when it is exposed to a mechanical shear stress and recovers its viscoelastic properties once back to the linear viscoelastic region. Thus, upon use, when the hydrogel composition is placed in the cylinder of a syringe, it flows under the effect of the shear stress applied via the piston and recovers its cohesive state once the stress is release, i.e. once it has been injected in the zone of treatment. The viscosity of the hydrogel composition is neither too low nor too high, thus allowing an effective lubrification of the joint without interference with the joint. The hydrogel composition of the composition was found to exhibit lubricating properties equivalent to those of hyaluronic acid (Ostenil^{®}).

The hydrogel composition of the present invention has suitable tribological properties. It is strong enough to resist the force of the joint for a period of at least two to three weeks.

The hydrogel composition of the present invention has been found to be able to release pain, even in absence of a pharmacological agent.

The hydrogel composition of the present invention has also been found to be able to suitably locally release a pharmacological agent over a prolonged period. Combinations of pharmacological agents may also be released. Local administration avoids the undesirable effects that may be encounter upon systemic administration of a pharmacological agent. The slow and prolonged release offered by the hydrogel composition of the present invention allows an effective amount of pharmacological agent to be delivered while preventing any toxicity (the amount of released pharmacological agent is below its toxicity threshold). Suitably, the hydrogel composition of the present invention may be able to reduce pain over a period of two to three weeks.

This combination of valuable properties was achieved as the result of the specific formulation of the hydrogel composition.

Thanks to all these properties, the hydrogel composition of the present invention is particularly suited for the treatment of articular disorders, in particular when the combination of a pharmacological action and a viscosupplementation is desired or required.

The hydrogel composition of the present invention comprises:
(a) a hydrogel made of a polymer network comprising:
   (i) chitosan;
   (ii) cyclodextrin polymers, said cyclodextrin polymers being water soluble cyclodextrin polymers or a mixture of water soluble cyclodextrin polymers and water insoluble cyclodextrin polymers wherein the ratio of the weight of water soluble cyclodextrin polymers to the weight of water insoluble cyclodextrin polymers is about 1:1;
      wherein the ratio of the weight of the chitosan to the total weight of the cyclodextrin polymers is about 2:1;
(b) optionally a pharmacological agent, preferably an analgesic agent, incorporated in the polymer network.

The hydrogel composition according to the present invention is based on a physical hydrogel formed by interactions between two polymers: a positively charged chitosan (cationic polymer) and cyclodextrin polymers (anionic polymer). The chitosan typically bears protonated amino groups on its glucosamine repeat units and/or the anionic cyclodextrin polymers typically bear carboxylate groups on their polycarboxylic crosslinks.

The term "about" means in the context of the present invention that the concerned value may be lower or higher by 10%, especially by 5%, in particular by 1%, than the indicated value. It encompasses the indicated value and values that may be lower or higher by 10%, especially by 5%, in particular by 1%, than the indicated value. As a matter of examples, when a range is said to vary from about X to about Y, it includes the range from X to Y and optionally values that may be lower by 10%, especially by 5%, in particular by 1%, than X and values that may be higher by 10%, especially by 5%, in particular by 1% than Y.

### Chitosan

Chitosan is a linear chain polymer that presents extended coil conformation in solution and that ensures the macromolecular architecture of the hydrogel and gives it the required rheological properties.

Chitosan is a biocompatible and biodegradable cationic polymer. It is a linear polysaccharide composed of randomly distributed β-1,4-linked D-glucosamine (deacetylated unit) and N-acetyl-D-glucosamine (acetylated unit). Chitosan may be produced by deacetylation of chitin which is the structural element of the exoskeleton of crustaceans or it may be extracted directly from fungi.

Chitosan may be characterized by its degree of deacetylation, which represents the percentage of deacetylated glucosamine units on its macromolecular chain. Chitosan suitable for use in the present invention has typically a degree of deacetylation ranging from 60 to 95%, preferably greater than or equal to 70% and preferably lower than 90% or lower or equal to 76%.

Chitosan is commercially available in the form of powder or as flakes of varying grains size.

Chitosan is insoluble in an aqueous medium having a pH greater than or equal to 4, greater than or equal to 4.5, in particular greater than or equal to 5. In contrast, chitosan dissolves in an acidic aqueous medium when the pH is lower than 4. When the aqueous solution is sufficiently acidic, the amine functions -NH₂ of the D-glucosamine repeat units of chitosan are protonated into ammonium functions -NH₃⁺. It should be noted that at a pH less than or equal to 5 and greater than or equal to 4, the amine functions of the chitosan will be partially protonated, but not in a number sufficient to dissolve the chitosan. The degree of protonation to be achieved to obtain solubilization also depends on the molecular weight and the degree of deacetylation of chitosan: if the degree of deacetylation is low, a higher ratio of amine functions will have to be protonated than if it is high due to the hydrophobicity of the acetyl-D-glucosamine repeat units. A chitosan having a degree of deacetylation (DDA) of 70% contains 4.04 mmol of amino groups per gram. The amino groups content raises 4.73 and 5.45 mmol per gram for chitosans presenting DDA of 80% and 90% respectively.

The molecular weight of the chitosan may range from 200 to 300 kDa. Chitosan suitable for use in the present invention has typically a molecular weight of 256 kDa.

In some embodiments, the hydrogel comprises from 1 to 3% by weight of chitosan relative to the total weight of the hydrogel, preferably the hydrogel comprises about 2% by weight of chitosan relative to the total weight of the hydrogel.

### Cyclodextrin polymers

The cyclodextrin polymer used in the invention plays the role of physical cross-linker in the hydrogel composition via the establishment of ionic bonds with chitosan chains network. Such ionic bonds stabilize the hydrogel structure and give its elastic behavior at rest, and can be broken when a strain is applied, offering shear thinning character, and instantaneously reform at rest, offering self-healing properties. The cyclodextrin polymer ensures cohesion and viscoelastic properties of the hydrogel that cannot dissolve spontaneously in the physiological medium and migrate. The cyclodextrin polymer also forms inclusion complex with the pharmacological agent and allows the pharmacological agent to be slowly and locally released. Cyclodextrin polymers are polymers deriving from cyclodextrins. Cyclodextrins are cyclic oligomers composed of 6, 7 or 8 α-(1,4)-linked glucopyranose units, respectively known as [alpha], [beta] and [gamma] cyclodextrin. The structure of cyclodextrin can be compared to a truncated cone, the external portion of which has hydrophilic properties, while the interior forms a hydrophobic cavity that is capable of reversibly forming inclusion complexes with molecules, preferably with hydrophobic molecules.

The cyclodextrin polymers of the present invention are typically obtained from the polycondensation of native cyclodextrins (alpha, beta, gamma cyclodextrins) or cyclodextrins derivatives (hydroxypropyl, methyl cyclodextrins) crosslinked with polycarboxylic acids (such as citric acid, or 1,2,3,4-butanetetracarboxylic acid).

Known cyclodextrin polymers obtained by the route mentioned above can be soluble or insoluble in water, depending on their structure.

Suitable cyclodextrin polymers for use in the present invention include water soluble cyclodextrin polymers and water insoluble cyclodextrin polymers.

Water soluble anionic cyclodextrin polymers and water insoluble anionic cyclodextrin polymers may be prepared by polycondensation reactions between (poly)carboxylic acids and native cyclodextrins (alpha, beta, gamma) or derivatives thereof (methyl, hydroxypropyl cyclodextrins) as disclosed in EP 1 165 621. Depending on the extension of the polycondensation reaction, the obtained polymers are either soluble or insoluble in water. The soluble form of these cyclodextrin polymers presents a hyperbranched structure forming globular objects of around 50 nanometers. The insoluble form is the result of an extended polymerization degree that provokes crosslinked structure and results in the water insoluble character. However, this insoluble form is highly hydrophilic and readily swells in water with swelling rate of up to 450-500% (meaning that insoluble cyclodextrin polymer may absorb water up to five times its own weight). The chemical structure of the soluble and insoluble forms is the same. The difference is the extension of the crosslinking rate that influences their molar mass. The cyclodextrin polymers as used in the present invention, notably obtained by the process disclosed in EP 1 165 621, are characterized by a high cyclodextrin content, e.g. from 40 to 65 % by weight relative to the total weight of the polymer and by a high number of anionic carboxylic functions, e.g. from 3 to 5 mmol per gram of cyclodextrin polymers.

Cyclodextrin polymers used in the present invention, and notably obtained by said process, have a molar mass of about 15 000-30 000 g/mol, notably 20 000-30 000 g/mol, preferably 20 000-25 000 g/mol. The hydrogel of the present invention is thus able to provide a lubricating effect (viscosupplementation) even in absence of a high molar mass polymer such as hyaluronic acid. The molar mass of the polymer, as disclosed herein, refers to the number average molar mass, calculated by means of size exclusion chromatography (SEC) or gel permeation chromatography (GPC).

Both soluble and insoluble cyclodextrin polymers are typically obtained in accordance with the process disclosed in EP 1 165 621. The mixture resulting from the polycondensation reaction between cyclodextrin and polycarboxylic acid at the solid state is suspended in water and then separated by filtration on a sintered glass funnel. The insoluble part is washed with water and dried, while the filtrate is dialyzed and then freeze dried to yield the soluble part as a powder. When preparing a hydrogel composition according to the invention, powders of soluble and insoluble forms are weighted and mixed in predetermined ratios.

Suitable cyclodextrin polymers for use in the present invention notably comprises from 3.5 to 4.5 mmoles/g of carboxylic acid functions, typically about 4 mmoles/g of carboxylic acid functions. The mole number of carboxylic functions per grams of cyclodextrin polymer may be measured by acid-base titration in the presence of colored indicators. The measurement method consists in dissolving a predetermined quantity of a cyclodextrin polymer in a given volume of water in the presence of a colored indicator, for example phenolphthalein (a few drops), then gradually adding to said solution (drop by drop), in particular using a graduated tube, a 0.1 moles per liter (mol/liter) solution of sodium hydroxide, with stirring (using a magnetic bar, for example) until the solution containing the colored indicator changes color. The number of moles of sodium hydroxide added then corresponds to the number of moles of carboxylic acid functions neutralized.

In some embodiments, the polymer network of the hydrogel comprises only water soluble cyclodextrin polymers as cyclodextrin polymers.

In some embodiments, the polymer network of the hydrogel comprises a mixture of water soluble cyclodextrin polymers and water insoluble cyclodextrin polymers as cyclodextrin polymers, the ratio of the weight of water soluble cyclodextrin polymers to the weight of water insoluble cyclodextrin polymers being 1:1.

In some embodiments, the hydrogel comprises from 1 to 3% by weight of cyclodextrin polymers relative to the total weight of the hydrogel, preferably the hydrogel comprises 1% by weight of cyclodextrin polymers relative to the total weight of the hydrogel.

In some embodiments, the hydrogel comprises up to 3% by weight of water soluble cyclodextrin polymers relative to the total weight of the hydrogel and no water insoluble cyclodextrin polymers, typically from 1 to 3% by weight of water soluble cyclodextrin polymers relative to the total weight of the hydrogel and no insoluble cyclodextrin polymers, preferably the hydrogel comprises 1% by weight of water soluble cyclodextrin polymers relative to the total weight of the hydrogel and no insoluble cyclodextrin polymers.

In some embodiments, the hydrogel comprises 0.5% by weight of water soluble cyclodextrin polymers and 0.5% by weight of water insoluble cyclodextrin polymers relative to the total weight of the hydrogel.

In some embodiments, the hydrogel comprises from 1% to 3% by weight of chitosan, from 1% to 3% by weight of cyclodextrin polymers (soluble and/or insoluble fractions of cyclodextrin polymers) and from 94 to 98% by weight of water (water which typically contains 1% by weight of acid).

### Hydrogel composition

In some embodiments, the hydrogel composition is free from pharmacological agent(s). In some embodiments, the hydrogel composition consists of a hydrogel as disclosed herein above.

In some embodiments, the hydrogel composition comprises from 95% to 99.9% by weight of a hydrogel, preferably from 99% to 99.5% by weight of a hydrogel and from 0.1 to 5% by weight of a pharmacological agent, preferably from 0.1 to 1% or from 0.5 to 1% by weight of a pharmacological agent, relative to the total weight of the composition. Preferably, the pharmacological agent is an analgesic agent.

In some embodiments, the hydrogel composition comprises 1% by weight of a pharmacological agent, preferably 1% by weight of an analgesic agent, relative to the total weight of the composition.

The pH of the hydrogel composition typically ranges from 3 to 5, preferably from 3.1 to 3.5 or from 4.2 to 4.5.

Examples of suitable compositions include the following compositions:

| | Hydrogel % w/w relative to the weight of hydrogel | | | Composition % w/w relative to weight of the composition |
|---|---|---|---|---|
| | Chitosan (% w/w) | Soluble cyclodextrin polymers (% w/w) | Insoluble cyclodextrin polymers (% w/w) | Pharmacological agent (% w/w) |
| Composition 1 | 2 | 1 | 0 | 0 to 5 or 0 to 1 or 0.1 to 1, preferably 1 |
| Composition 2 | 2 | 0.5 | 0.5 | 0 to 5 or 0 to 1 or 0.1 to 1, preferably 1 |

### Preparation of the hydrogel composition

The hydrogel composition that comprises a pharmacological agent may be suitably prepared by adding the pharmacological agent upon preparation of the hydrogel.

The hydrogel may be suitably prepared in accordance with the method disclosed in WO WO2017/001808. In the disclosed method, cyclodextrin polymers (soluble and insoluble cyclodextrin polymers) and chitosan are provided as powders. The term "powder" as used herein designates a solid substance that is reduced to small grains at ambient temperature. The powder of cyclodextrin polymers (mixed insoluble and soluble forms, or soluble form only) and chitosan are dry mixed to form a mixture of powders. The obtained mixture of powders is suspended in an aqueous medium having a pH that enables soluble cyclodextrin polymer to be dissolved without dissolving the chitosan (neither the insoluble cyclodextrin polymer), for example at a pH greater than or equal to 4, at a pH greater than or equal to 4.5, or even greater than or equal to 5, and then the resulting suspension is acidified to form a bulk viscoelastic hydrogel under strong agitation.

Based on this method, the hydrogel composition of the present invention may be prepared as described in details herein below. The hydrogel composition may be prepared at room temperature.

Cyclodextrin polymers (soluble and insoluble cyclodextrin polymers) and chitosan are provided as powders. The powders may be sterilized before use.

### Preparation of the powders

Chitosan and cyclodextrin polymers can be used as raw powders, i.e. as uncalibrated particles. The cyclodextrin polymer powders and/or the chitosan powder may be sieved before being used. Sieving may be suitably performed through a sieve with a mesh with a size less than or equal to 500 µm, for example, less than or equal to 300 µm, or even less than or equal to 200 µm, in particular less than or equal to 150 µm, more particularly less than or equal to 125 µm. Chitosan powder and cyclodextrin polymer powders may be sieved with the aid of a vibrating sieve, for example a vibrating sieve as sold by Fritsch.

In some embodiments, the cyclodextrin polymer powders and/or the chitosan powder may be milled prior to being sieved, for example, with the aid of a Pulverisette 14^{®} machine sold by Fritsch and provided with a 120 µm sieve. Milling can reduce the size of the solid particles and form smaller fragments. The control of the particles size of chitosan and insoluble cyclodextrin polymer may be important in the process of preparation of the hydrogel. Indeed, small particles are more readily suspended in water, and allow fast dissolution of chitosan after the acidification of the suspension. These parameters may be important not only for the gel formation time, but also for the homogeneity of the bulk hydrogel (absence of lump in the hydrogel).

In some embodiments, the water soluble cyclodextrin polymer powder and the water insoluble cyclodextrin polymer powder, when present, are milled in a mortar and sieved through a 125 µm sieve. The chitosan is milled separately prior to being sieved, for example, with the aid of a Pulverisette 14^{®} machine sold by Fritsch and provided with a 120 µm sieve. The obtained chitosan powder is then sieved again with a 125 µm sieve.

In some embodiments, the cyclodextrin polymer powders and/or the chitosan powder are milled to obtain particles having an average size lower than 125 microns. The size distribution of the particles of the chitosan powder and/or the cyclodextrin polymer powders or of the mixture of powders of chitosan and cyclodextrin polymer powder(s) may be measured using a Mastersizer S (Malvern Instruments, Orsay, France) using a 300 mm lens. The sample is dispersed in the dry state with compressed air at 4 bars.

### Addition of the pharmacological agent

In some embodiments, the hydrogel composition comprises a pharmacological agent incorporated in the polymer network.

In those embodiments, the pharmacological agent is added by wet granulation to the cyclodextrin polymer powder(s). Since the pharmacological agent can react with the chitosan amino groups, this process prevents any interaction which could affect the hydrogel formation and stability. The pharmacological agent may be added to the water soluble cyclodextrin polymer powder or to a mixture of water soluble and water insoluble cyclodextrin polymer powders that may have been milled and/or sieved prior to use. The pharmacological agent and the cyclodextrin polymer powder(s) are provided in the proportions indicated herein above. Wet granulation may be performed by any method well-known in the art. For instance, the cyclodextrin polymer powder(s) and the pharmacological agent may be placed in a mortar. Then water is added, for instance in a 4:1 proportion (w/w) and the resulting mixture is stirred. Then, the mixture is placed in an oven at 60°C for 1h30. The obtained powder may then milled and sieved to obtain particles having a size lower than 125 microns.

The cyclodextrin polymer powder(s) comprising the pharmacological agent and the chitosan powder are then dry mixed. The dry mixing may be suitably be made using a mixer mill.

In some embodiments, the resulting mixture may be sieved or co-milled and sieved. Sieving may be performed as disclosed above.

In some embodiments, the cyclodextrin polymer powder(s) comprising the pharmacological agent and/or the chitosan powder may be co-milled, for instance they can be co-milled manually in a mortar or with a Mixer Mill MM400 sold by Restch, Steinbach, Germany. The term "co-milling" refers to the simultaneous milling of at least two different powders. It has been observed that co-milling and/or milling followed by mixing can reduce the size of the solid particles of the powders, and can also provide intimate mixing between the powders (and thus a better contact surface area between the cyclodextrin polymers and the chitosan favorizing the electrostatic interactions). Milling promotes the homogeneity of the aqueous dispersion which is formed with these powders in the next step of the method. The reproducibility in the formation of the hydrogel is also increased, as well as the homogeneity of the hydrogel which presents no visible lumps.

### Preparation of a suspension and acidification to form a hydrogel composition

After being dry mixed and optionally sieved or milled and sieved, the obtained mixture of cyclodextrin polymer powder(s) optionally comprising the pharmacological agent on the one hand, and chitosan powder on the other hand, is then suspended in an aqueous medium having a pH that enables the cyclodextrin polymer to be dissolved without dissolving the chitosan. The suspension is preferably stirred. The pH of the aqueous medium suitably ranges from 5 to 6, preferably from 5.5 to 6. pH values indicated in the present text are measured using a pH meter at ambient temperature (i.e. 20 - 25°C). The expression "without dissolving the chitosan" as used herein means that no amine function of the chitosan or an insufficient number of amine functions of the chitosan is protonated in the aqueous medium to cause dissolution of the chitosan powder. The aqueous medium is preferably water (for example, distilled water or ultrapure water). In general, at least 0.5%, or even at least 1% by volume of an acid may be added to the aqueous medium relative to the total volume of said aqueous medium.

The resulting suspension is then acidified in order to form the hydrogel composition. Upon addition of the acid, the pH of the suspension decreases, particularly to a pH lower than or equal to 5, more particularly lower than or equal to 3, in particular lower than or equal to 4, which causes the solubilization of chitosan by protonation of its amines into ammonium functions. The chitosan macromolecule unfolds under the effect of intramolecular electrostatic repulsions between the ammonium groups formed and reaches an extended coil conformation that occupies the entire volume of the aqueous medium once the "critical" concentration C* (generally expressed in g/cm³) is reached. This critical concentration C* corresponds to the concentration above which the polymer coils overlap. Simultaneously, ionic bonds are formed between the ammonium groups of the chitosan and the anionic functions of the cyclodextrin polymer. Then, once the amine functions are protonated, the pH of the hydrogel rises to stabilize, preferably to a pH greater than or equal to 4, even more preferably to a pH greater than or equal to 4.5, in particular to a pH greater than or equal to 5.

Suitable acids that may be added to the suspension to form the hydrogel composition include, but are not limited to, acetic acid, in particular glacial acetic acid, formic acid, tartaric acid, salicylic acid, glutamic acid, propanoic acid, hydrochloric acid, citric acid, lactic acid and mixtures thereof. Preferred suitable acids include acetic acid, hydrochloric acid, lactic acid and mixtures thereof. More preferred suitable acids include lactic acid and hydrochloric acid, in particular lactic acid.

It has been observed that the firmness and stability of the injected hydrogel composition are better with these acids, in particular with lactic acid. In addition, very diluted solutions of hydrochloric and lactic acids have the advantage of being odorless, compared with acetic acid. The lactic acid added may be an aqueous solution comprising at least 85% by weight of pure lactic acid relative to the volume of said aqueous solution.

Typically, the acidification is performed by adding an aqueous composition comprising at least 1% by weight of lactic acid. Additionally, the acidification step has to be accompanied with vigorous stirring. The shear forces promote the fast dissolution of chitosan and the hydrogel formation.

The present invention also relates to a method for providing a hydrogel composition as described herein comprising the following steps:
(i) providing water soluble cyclodextrin polymer powder, water insoluble cyclodextrin polymer powder when applicable and chitosan powder;
(ii) adding by wet granulation a pharmacological agent to the water soluble cyclodextrin polymer powder or to the mixture of water soluble and water insoluble cyclodextrin polymer powders;
(iii) dry mixing the powder obtained in step (ii) with chitosan powder in order to form a mixture of powders;
(iv) optionally sieving or co-milling and sieving the mixture of powders obtained in step (iii);
(v) suspending the mixture of powders obtained in step (iv) in an aqueous medium having a pH that enables the water soluble cyclodextrin polymer to be dissolved without dissolving the chitosan, for example at a pH greater than or equal to 4, at a pH greater than or equal to 4.5, or even greater than or equal to 5;
(vi) acidifying the suspension obtained from step (v) in order to form the hydrogel composition.

The steps of the method are as disclosed in details herein above.

Stated differently, the present invention also relates to a hydrogel composition susceptible of being obtained by a method comprising the following steps:
(i) providing water soluble cyclodextrin polymer powder, water insoluble cyclodextrin polymer powder when applicable and chitosan powder;
(ii) adding by wet granulation a pharmacological agent to the water soluble cyclodextrin polymer powder or to the mixture of water soluble and water insoluble cyclodextrin polymer powders;
(iii) dry mixing the powder obtained in step (ii) with chitosan powder in order to form a mixture of powders;
(iv) optionally sieving or co-milling and sieving the mixture of powders obtained in step (iii);
(v) suspending the mixture of powders obtained in step (iv) in an aqueous medium having a pH that enables the water soluble cyclodextrin polymer to be dissolved without dissolving the chitosan, for example at a pH greater than or equal to 4, at a pH greater than or equal to 4.5, or even greater than or equal to 5;
(vi) acidifying the suspension obtained from step (v) in order to form the hydrogel composition.

Step (vi) is typically performed under stirring with high shear rate.

The disclosed method may be implemented with a device comprising a first syringe receiving the chitosan, the cyclodextrin polymers powders and the pharmacological agent and a second syringe receiving a predetermined volume of an acidified aqueous medium, said device including means for placing the first and second syringe in fluid communication, in a manner such as to allow the acidified aqueous mixture to be combined with the mixture comprising the chitosan, the cyclodextrin polymers powders and the pharmacological agent. Said means for fluid communication may optionally be detachable from the first syringe and the second syringe. The first and second syringes may be in fluid communication at their injection ends via means for fluid communication (for example Luer Lock), which allow the acidified aqueous medium to be introduced into the first syringe with the aid of the piston of the second syringe, and vice versa via the piston of the first syringe. The pistons are actuated multiple times (about 50-70 repetitions) until a hydrogel is formed; the shear forces that are generated promote dissolution of the chitosan powder and the ionic interactions with the anionic cyclodextrin polymer in order to cause a stable, firm hydrogel to be readily formed.

### Administration of the hydrogel composition

The hydrogel composition may be suitably administered by injection, in particular by intra-articular injection. The injection may be made with a device comprising the hydrogel. Said device is preferably a syringe comprising at least one reservoir filled with the hydrogel composition, typically filled with a predetermined volume of the hydrogel composition.

In some embodiments, the device for administering the hydrogel composition comprises a first syringe containing the mixture comprising the chitosan, the cyclodextrin polymer powders and the pharmacological agent, when present, preferably comprising a suspension as described herein comprising the chitosan, the cyclodextrin polymer powder(s) and the pharmacological agent when present and a second syringe containing a predetermined volume of an acidified aqueous medium, said device including means for placing the first and second syringe in fluid communication, in a manner that allows the acidified aqueous medium to be combined with the mixture comprising the chitosan, the cyclodextrin polymer powders and the pharmacological agent, when present. Once the hydrogel composition is formed as disclosed herein above, the syringe comprising the hydrogel composition may be fitted with a needle and the hydrogel composition may be injected.

The present disclosure also describes a non-claimed method for the treatment of articular disorders, in particular when the combination of a pharmacological action and a viscosupplementation is desired or required, which comprises the administration to an individual in need thereof, of an effective dose of a hydrogel composition as disclosed herein. The administration of the hydrogel composition is typically performed by injection, in particular by intra-articular injection.

The effective dose of the hydrogel composition varies as a function of numerous parameters such as, for example, the weight, age, sex, the state of progress of the disorder to be treated and the sensitivity of the individual to be treated.

The present invention also relates to a hydrogel composition for use in the manufacture of a medicament for use in the treatment of articular disorders, in particular when the combination of a pharmacological action and a viscosupplementation is desired or required.

Embodiments of the present invention will now be described by way of the following examples which are provided for illustrative purposes only, and not intended to limit the scope of the disclosure.

### EXAMPLES

### Abbreviations

- CHT: chitosan
- PCDs: water soluble anionic cyclodextrin polymers
- PCDi: water insoluble anionic cyclodextrin polymers
- NaNX: sodium naproxen
- βCD: Beta Cyclodextrin

### Example 1: Stability of hydrogels

The hydrogels were prepared in accordance with the method disclosed in WO WO2017/001808. The cohesion of the following formulations were assessed (Table 1).

**Table 1: CHT:PCDs:PCDi formulations**

| Formulation CHT/PCDs/PCDi | CHT (%w/w) | PCDs (%w/w) | PCDi (%w/w) |
|---|---|---|---|
| 1.5 :1.5:0 | 1.5 | 1.5 | 0 |
| 1.5 :0.75 :0.75 | 1.5 | 0.75 | 0.75 |
| **2 :1 :0** | **2** | **1** | **0** |
| **2 :0.5 :0.5** | **2** | **0.5** | **0.5** |

After preparing the hydrogels, hydrogels cohesion was evaluated by injection into a vial and inverting it. One mL of hydrogel was injected into a vial at 37°C and the flow resistance was visually assessed over time.

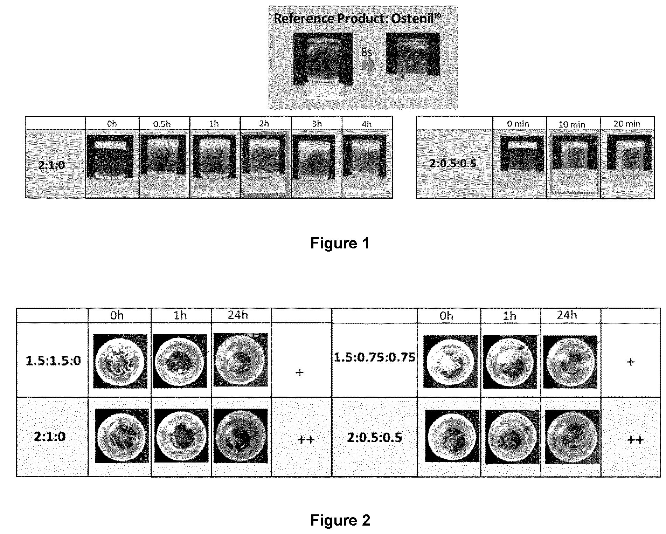

A hyaluronic acid-based product (R.P.) called Ostenil ^{®} was used as a control and tested at the same time (Figure 1).

All CHT/PCDs/PCDi tested formulations formed a hydrogel.

The R.P. flowing resistance was lower (less than 8 seconds) compared to the one of CHT/PCDs/PCDi formulations.

A variable cohesion was observed between the samples. The 2:1:0 formulation showed a high flow resistance (>2h) compared to the 1.5:1.5:0 (2h) formulation. Addition of PCDi led to a decreased flow resistance reflecting a weaker hydrogel cohesion (1.5:0.75:0.75 and 2:0.5:0.5).

The hydrogels were then injected with an 18G needle into a buffer solution (Buffer Phosphate Saline PBS, pH 7.4, 80 rpm, at 37°C) to test the hydrogel cohesion in a media that mimicked physiological pH. Since R.P. is a hyaluronic acid-based solution, it does not form a cohesive product after injection (it forms a solution with PBS, results not presented).

Results are presented on Figure 2. A cord-like structure was observed with all samples. However, a stronger cohesion was observed for 2:1:0 and 2:0.5:0.5 hydrogels over 24 hours vs. 1.5:0.75:0.75 and 2:0.5:0.5 hydrogels. More particularly, 2:0.5:0.5 hydrogel presented the highest stability.

1.5:1.5:0 and 1.5:0.75:0.75 hydrogels showed a weak cohesion over 24h. Indeed, formulation 1.5:1.5:0 lost its structure by shrinking after 1 hour, while formulation 1.5:0.75:0.75 partially lost the cord-like structure and formed a lump.

In conclusion, a stronger cohesion is observed for hydrogels in accordance with the present invention, i.e. the 2:1:0 and 2:0.5:0.5 hydrogels. The hydrogels in accordance with the present invention are stable.

Despite the higher flow resistance of the 2:1:0 formulation, PCDi addition improves the structural stability in formulation 2:0.5:0.5.

### Example 2: Viscoelastic properties of hydrogels

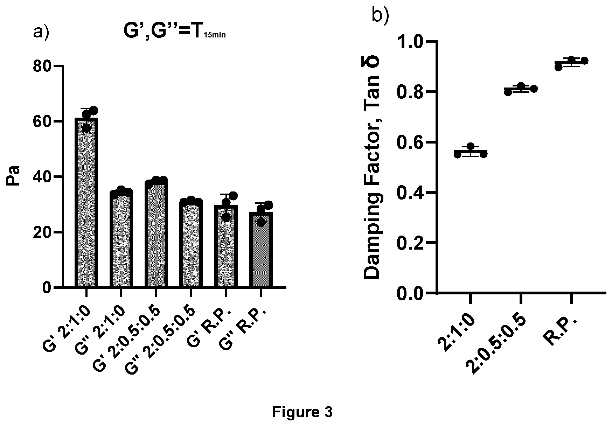

The study of G' and G" (Pa) was carried out by rheological measurement using a rheometer in the linear viscoelastic region (γ=1%, frequency=10rad/sec, 37°C). The results are presented in table 2 below and on Figure 3.

As shown in Table 2, G' is higher than G" (G'>G") in CHT/PCDs/PCDi hydrogels, which demonstrates the formation of a viscoelastic solid.

It can be observed that CHT/PCDs/PCDi hydrogels had a superior viscoelastic behavior compared to Ostenil^{®} (referred to as R.P.). Indeed, R.P. has a behavior corresponding to a viscoelastic solid, but the difference between G' and G" being quite low, it can be concluded that it exhibits a weaker cohesion.

The Tan δ (Table 2), also known as the damping factor, is a parameter that helps to differentiate the internal energy (or force of interaction) of viscoelastic solids. In rheology, the values of Tan δ is calculated from the ratio G"/G'; thus, tan d > 100:1 = 100 is considered as an ideal viscous liquid; 0,01< tan d < 100 is considered as viscoelastic behavior; tan d < 1:100 = 0,01 is considered as ideal elastic solid.

In the case of the CHT/PCDs/PCDi formulations, low values of tan δ (<0.82) were observed compared to the R.P. (0.92). This is proof of a higher solid elastic character of the hydrogel. Concerning the CHT/PCDs formulations, similar tan δ values are observed (0.56) as well as for the formulations with CHT/PCDs/PCDi (0.82) (Figure 3).

**Table 2: Values of the modulus G' and G" and Tan δ**

| | Storage Modulus (G') | Loss Modulus (G") | Tanδ |
|---|---|---|---|
| **2:1:0** | **61.23** | **34.37** | **0.56** |
| **2:0.5:0.5** | **38.16** | **30.97** | **0.82** |
| R.P. | 29.66 | 27.17 | 0.92 |

In conclusion, the hydrogels CHT:PCDs/PCDi compositions of table 2 behave as solid viscoelastic materials while RP behaves as a viscous liquid.

### Example 3: Injectability of hydrogels (Study of viscosity versus shear rate)

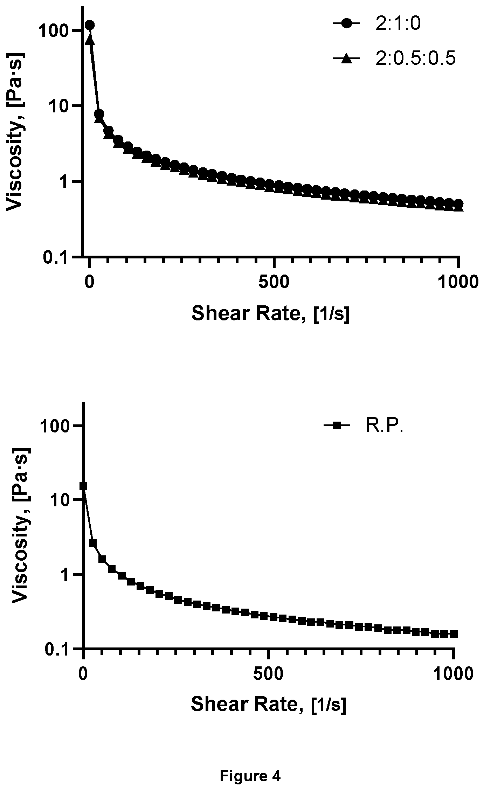

A viscosity evolution versus the shear rate sweep by rheology measurement was made to study the hydrogel injectability (25°C). CHT/PCDs/PCDi hydrogels were tested as well as R.P to see the impact of PCD addition in the formulation. The R.P. has a lower resistance (0.16 Pa.s at 1000 s⁻¹) to flow whereas the CHT/PCDs/PCDi hydrogels had a slight higher resistance (0.47-0.49 Pa.s at 1000 s⁻¹) (Figure 4; table 3). It can be concluded that CHT/PCDs/PCDi and R.P are non-Newtonian and have a shear-thinning behavior.

These characteristics prove the hydrogel injectability.

**Table 3: Viscosity values (Pa.s) at 0 s⁻¹ and 1000 s⁻¹**

| | Viscosity 0 s⁻¹ (Pa.s) | Viscosity 1000 s ⁻¹(Pa.s) |
|---|---|---|
| **2:1:0** | **112.85** | **0.49** |
| **2:0.5:0.5** | **76.33** | **0.47** |
| R.P. | 15.49 | 0.16 |

### Example 4: Self-healing of hydrogels (G' modulus recovery versus deformation)

The self-healing property is the capacity of some hydrogels to reorganize their structure after the internal bonds have been broken under deformation induced by mechanical stress. The recovery of the hydrogels under different shear stress was evaluated to determine the recovery rate of the hydrogels. Weak (γ=1%) and strong (γ=500%) stress cycles at 25°C were applied to all samples.

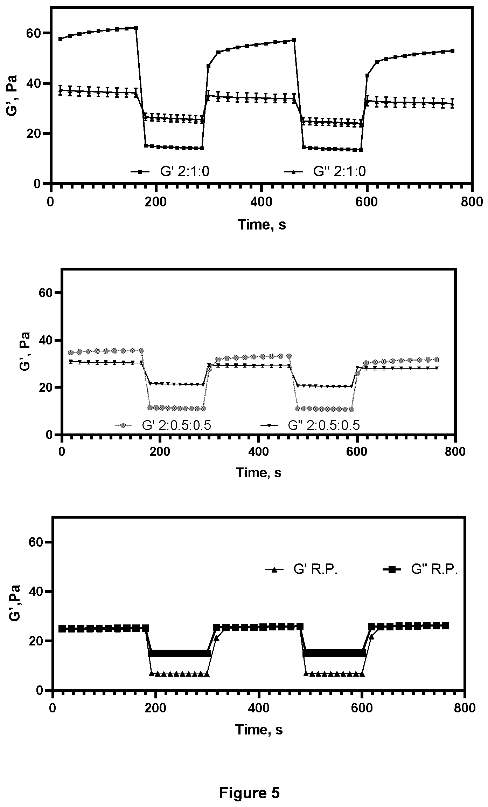

Figure 5 shows the G' and G" moduli of CHT/PCDs/PCDi hydrogels as well as R.P in function of applied shear amplitudes cycles of 1% and 500%. At low shear amplitude G' is superior to G" and the gels has solid viscoelastic behavior. The impact of high stress triggers the decrease of G' modulus below the value of G", that displays flowing behavior. G' recovery is then observed once back to low shear amplitude. CHT/PCDs/PCDi and R.P had a G' recovery rate above 90%. The explanation is that under low shear amplitude the chitosan and PCDs and PCDi are in interactions by ionic bondings (they form a polyelectrolyte complex). Under high shear amplitude, the ionic interactions are broken and both polymers recover their mobility and the system flows (G"> G'). In a short time after low shear amplitude is then applied again, ionic bondings are immediately recreated and the initial parameters are recovered.

Therefore, CHT/PCDs/PCDi formulations can be considered a self-healing hydrogel.

### Example 5: Formulation of hydrogels with a pharmacological agent

### Preparation of a hydrogel composition comprising a pharmacological agent

A concentration of 1% w/w of NaNX was added to the formulation by PCDs/PCDi/NaNX wet granulation as described previously.

### Rheological evaluation of hydrogel compositions comprising a pharmacological agent

The evaluation was performed as disclosed herein above in connection with the hydrogels.

### G' and G" evaluation over time

The rheological evaluation was done to evaluate any changes after NaNX addition.

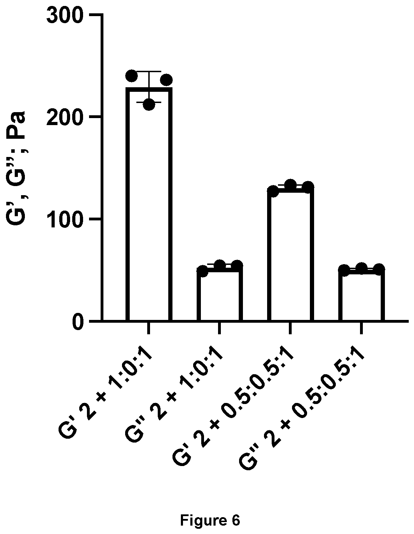

Figure 6 shows the formation of a hydrogel in both cases (G'>G"). Indeed, the inclusion of NaNX triggered the increase of the G' modulus. A value of G' and G" modulus of 230 Pa and 55 Pa respectively are observed in the formulations 2+1:0:1 and 130 Pa and 50 Pa respectively in the formulations 2+0.5:0.5:1 (table 4).

The Tan δ values were compared as well, formulations with a pharmacological agent (<0.39) formed a stronger hydrogel compared to formulations without pharmacological agent (0.50-0.82).

In conclusion, the formation of a stronger hydrogel is obtained after wet granulation between PCD and NaNX. This test proved that the addition of NaNX allows maintaining, the viscoelastic properties of CHT+PCDs:PCDi hydrogels.

**Table 4: Evaluation test values of G' and G"**

| CHT+PCDs:PCDi:NaNX | Storage Modulus (G') | Loss Modulus (G") | Tanδ |
|---|---|---|---|
| 2+1:0:1 | 230 | 55 | 0.23 |
| 2+0.5:0.5:1 | 130 | 50 | 0.39 |

### Study of viscosity versus shear rate

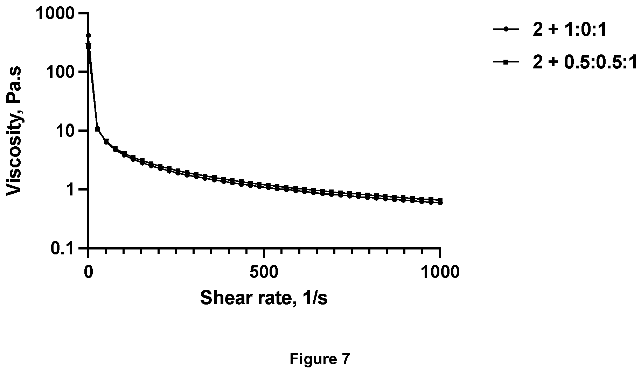

Figure 7 shows the viscosity study of hydrogel compositions containing NaNX. A significant increase of viscosity at 0.01 s-1 is observed with values of 415 and 268 (table 5). A fourfold increase over initial values for hydrogels without NaNX is shown.

The results show that the addition of NaNX allows maintaining the non-Newtonian and shear-thinning behavior of CHT+PCDs:PCDi hydrogels. As previously stated, the shear-thinning property is related to the hydrogel injectability.

**Table 5: Viscosity values (Pa.s) at 0 s⁻¹ and 1000 s⁻¹**

| CHT+PCDs:PCD:NaNX | Viscosity 0 s⁻¹ (Pa.s) | Viscosity 1000 s ⁻¹ (Pa.s) |
|---|---|---|
| 2+1:0:1 | 415 | 0.59 |
| 2+0.5:0.5:1 | 268 | 0.65 |

### Recovery

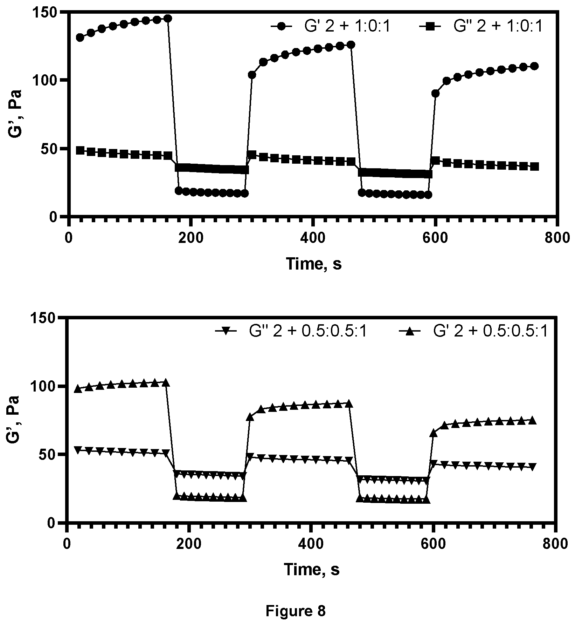

The same experimental parameters as described previously were used to perform this test. Similar behavior is observed and recovery of G' around 90% is obtained. As mentioned previously, this test displays the loss of the polymer network structure at high shear amplitude as well as the reorganization of new ionic bonds once back to a low stress. (Figure 8)

### Study of the release in vitro

The release study was performed to assess the kinetics of NaNX release. A quantity of 0.8g of the hydrogel composition (see table 6) was injected into a 1 kDa regenerated cellulose dialysis membrane (Spectra/Por^{®}, Spectrum Laboratories, Inc). Then, the sample was tested in a USP 1 dissolution system (Agilent, France) in 500 mL of phosphate buffer saline (PBS) at 37°C. All tests were performed in triplicate.

All samples were analyzed by ultra-performance liquid chromatography coupled to a diode array detector (Shimadzu Nexera-i LC-2040 3D plus). The mobile phase was composed of an acetonitrile/orthophosphoric acid pH 2.25 (65:35) mixture injected at a 1 mL/min flow rate into a C18 column (4 * 250 mm) stabilized at 25°C. The injection volume was fixed at 20 µL and the detection wavelength was 225 nm. A retention time of 4.2 minutes was established to calculate the area under the curve (AUC).

**Table 6: wt% of naproxen released**

| | wt% Released over 36hr |
|---|---|
| 2 + 1:0:1 | 86.0 ± 1.2 |
| 2 + 0.5:0.5:1 | 78.1 ± 2.4 |

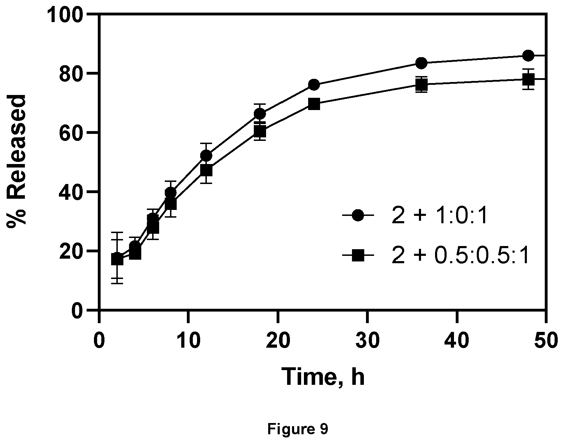

First, a similar release profile is observed for both cases; indeed, a release plateau is reached after 36 hours. Some slight differences were as well observed between both formulations, a greater drug quantity was released in the 2+1:0:1 hydrogel (86.0wt%) whereas 2+0.5:0.5:1 hydrogel released a 78.1wt% (Figure 9).

### Example 6: Assessment of cytotoxicity

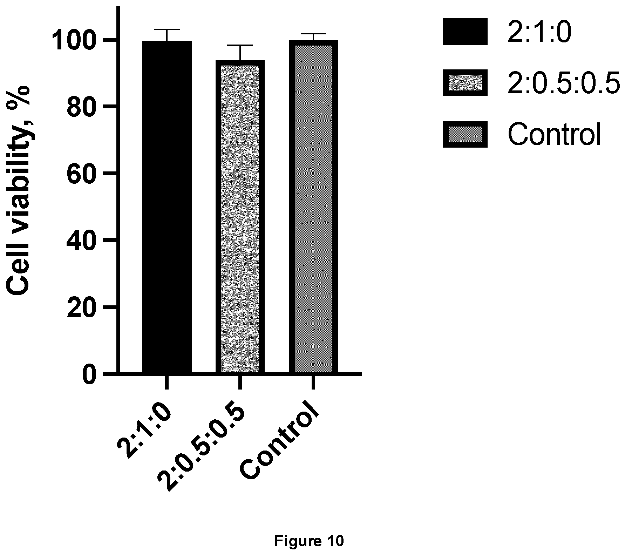

The cytotoxicity of the hydrogel formulations 2:0.5:0.5 and 2:1:0 (see table 1) was evaluated by the extraction method (indirect contact) according to the ISO 10993-5 standard using the cell line of NIH/3T3 fibroblast cells. The hydrogels were preconditioned beforehand in the MEM-α culture medium at 37° C. and 80 rpm for 2 h. The culture medium was removed and a concentration of 200mg of hydrogel / 1mL medium was added. On the other hand, NIH3T3 cells were seeded in a cell culture plate to form a cell carpet. The number of cells seeded was 4×103 cells per well in 100 µL of the complete medium and incubated at 37° C. under an atmosphere of 5% CO2 for 24 h. After incubation, each extraction medium (n=2) was sterilized using a 0.22 µm filter. Then, the cell layer culture medium was replaced with 100 µL of the sterile extraction medium. The cells were incubated for an additional 24 h, at 37° C. under an atmosphere of 5% CO2. Finally, cell viability was evaluated by fluorometry with the AlamarBlue^{®} reagent (Uptima, Interchim, France). Fluorescence reading was measured at 530 nm as excitation wavelength and 590 nm as emission wavelength. Cellular metabolic activity was expressed as the percentage of fluorescence intensity relative to the control value (Figure10).

Figure 10 shows the percentage of cell survival following exposure to the hydrogels with the culture medium. High cell viability was observed for both formulations compared to the control. This test proves the safety of the administration of this hydrogel on human beings.

### Example 7: Tribological study

A tribology study was made on a 2:0.5:0.5 hydrogel (see table 1) using a Tribo-Rheometer MCR 301 connected to a T-PID 44 tribology cell and a SCFE7 measuring accessory (Anton Paar, Les Ulis, France). An accessory of 3 pins shaft and a PTFE disk were used to determine the lubrication activity of hydrogels and R.P. A deflection angle of 50 mrad, angular frequency of 6rad/s and a normal force of 45 N were applied. A pre-stabilization was applied to depressurize the system before starting each cycle. This protocol consisted of a deflection angle of 0.014 mrad, a angular frequency of 1 rad/s and a normal force of 15N for 5 min.

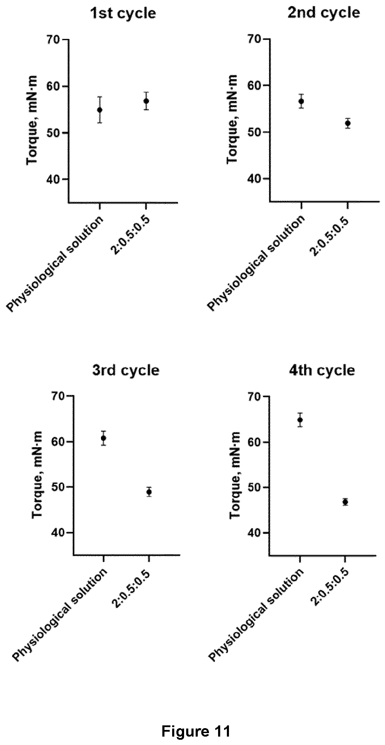

Torque was measured for 30 minutes over 4 cycles. At the same time, a sterilized sample of said formulation was tested and results are shown in Figure 11.

The hydrogels 2:0.5:0.5 were also compared to a physiological solution (NaCl 0.9%) as a negative control. In the first cycle, negative control and non-sterilized samples have a comparable behavior Next, negative control tend to increase over time whereas formulation 2:0.5:0.5 decreased over time, proving the lubrication activity of formulation 2:0.5:0.5.

### Example 8: Sterilization of hydrogel

Ethylene Oxyde (EtO) sterilization and gamma irradiation were used to sterilize the powder of CHT and PCD respectively. The sterilization procedure was carried out in accordance with ISO 11135 et ISO 11137 standards concerning the sterilization of health products. Sterilization by EtO consisted of 3 parts: Pre-packaging, Sterilization (exposure to EtO for 3 hours) and Aeration. EtO gas is eliminated by using air changes in the sterilization chamber to remove the remaining EtO particles.

Gamma irradiation sterilization was performed using a source of cobalt-60 (60Co) radiation at a dose of 40 kGy.

The impact of sterilization on the hydrogel were performed for the formulations CHT/PCDs/PCDi 2:0.5:0.5 and CHT+PCDs/PCDi/NaNX 2+0.5:0.5:1. The evaluation consisted of the study of viscoelastic properties (G' and G") and cytocompatibility (only for formulation without NaNX).

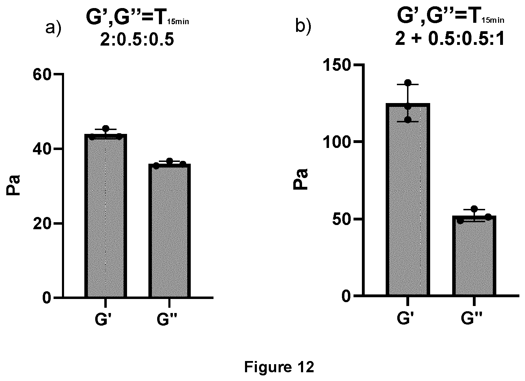

Results obtained are shown in figure 12 and table 7. The viscoelastic properties were evaluated as described previously (Figure 12, table 7), the results showed a prevalence of elastic modulus over viscous modulus, which confirms the formation of hydrogel formation (G'>G").

**Table 7: Values of elastic and viscous moduli (Pa) before and after sterilization**

| **Formulation** | **Before Sterilization** | | | **After Sterilization** | | |
|---|---|---|---|---|---|---|
| | **G' (Pa)** | **G" (Pa)** | **Tanδ** | **G' (Pa)** | **G" (Pa)** | **Tanδ** |
| **CHT/PCDs/PCDi 2 :0.5 :0.5** | 49 | 36 | 0.75 | 50 | 39 | 0.84 |
| **CHT+ PCDs/PCDi/NaNX 2+0.5 :0.5 :1** | 130 | 50 | 0.39 | 125 | 52 | 0.41 |

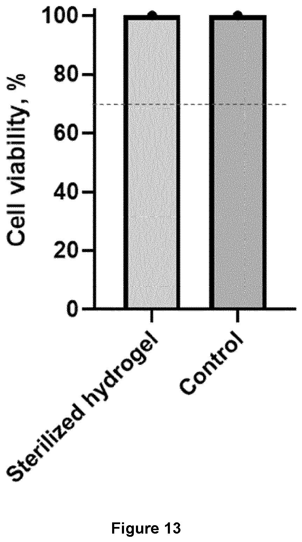

Then, a cytocompatibility study was performed according to ISO 10993-5 as described previously. No difference of cell viability was observed after sterilization (Figure 13). Indeed, a value of cell viability of 100% (over 93.8% cell viability before sterilization) was obtained, proving its cytocompatibility.

### Example 9: preclinical studies of hydrogel

A total of twenty male Wistar rats (6-week-old) with an average weight of 221.6 g (range from 193 to 267 g) per group were used. Animals were housed in a temperature-controlled room (22 +/- 1°C), with a 12-12h light-dark cycle. After reception, each animal was placed into individual cage, food and water were given ad libitum. One left Temporo Mandibular Joint (TMJ) injection of 0.5 mg of monosodium iodoacetate (MIA, Sigma, Saint Louis, USA) dissolved into 50 µL of saline solution was done under general anesthesia to induce a TMJ osteoarthritis. The solution was injected into the upper compartment of the left TMJ with a 26-gauge needle using anatomical landmarks. The same injection technique was used in both joints for the therapeutic arm on 2 days post-MIA injection (2:0.5:0.5 and R.P.)

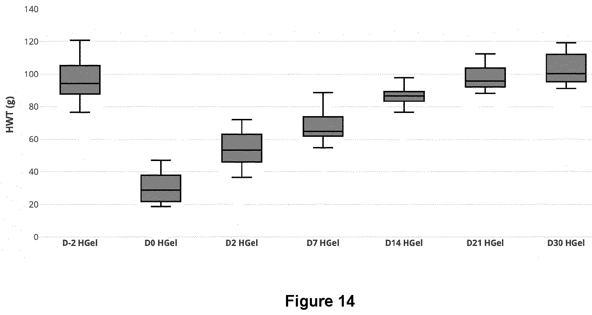

Figure 14: The pain experienced by the rat after intra-TMJ injection of chemical agent was measured by a Von Frey aesthesiometer before the injection of the therapeutic agent (days - 2, 0) and on days 2, 7, 14, 21 and 30 post-injection. Briefly, the hard-plastic tip was used, on the left side, to stimulate the midpoint of the connection between the eyes and ears of the rat's head and face. In the process of the experimental operation, the mechanical stimulation intensity increased, and in turn, rat behaviour was observed at the same time. When reactions such as rubbing the mouth or scratching the head or head withdrawal were observed, the stimulus intensity (g) was recorded as head withdrawal threshold (HWT), which was defined as the lowest pressure on TMJ to induce nociception. Afterwards, the rat was allowed to rest some minutes, the same manipulation was then applied on the right side. The HWT was calculated as a mean value per joint of all tested rats/group.

Whereas the HWT on the (left) side significantly decreased 2 days after MIA injection, the HWT returned to an original level from D14 after 2:0.5:0.5 Hydrogel injection, emphasizing the lubricant effect of Hydrogel. Compared with the R.P, HWT is significantly higher in the hydrogel group from day 14 to day 30.

## Claims

1. A hydrogel composition comprising:
(a) a hydrogel made of a polymer network comprising:
(i) chitosan;
(ii) Anionic cyclodextrin polymers, said anionic cyclodextrin polymers being water soluble anionic cyclodextrin polymers or a mixture of water soluble anionic cyclodextrin polymers and water insoluble anionic cyclodextrin polymers (CDPi) wherein the ratio of the weight of water soluble anionic cyclodextrin polymers to the weight of water insoluble anionic cyclodextrin polymers is about 1:1;
wherein the ratio of the weight of the chitosan to the total weight of the anionic cyclodextrin polymers is about 2:1, the term "about" meaning that the concerned value is lower or higher by 10%, especially by 5%, in particular by 1%, than the indicated value; and
(b) optionally a pharmacological agent incorporated in the polymer network,
for use in the treatment of articular disorders.

2. The hydrogel composition for use according to claim 1 wherein the pharmacological agent is present and is an analgesic agent.

3. The hydrogel composition for use according to claim 2 wherein the analgesic agent is selected from the group consisting of opioid analgesic agents, such as codeine, oxycodone and hydromorphone, and nonsteroidal anti-inflammatory agents, such as naproxen, ibuprofen diflunisal and ketorolac.

4. The hydrogel composition for use according to any one of the preceding claims wherein the articular disorders are osteoarthritis, osteoarthrosis, rheumatic disorders and degenerative meniscal lesions.

5. The hydrogel composition for use according to any one of the preceding claims wherein the hydrogel composition is administered by intra-articular injection.

6. The hydrogel composition for use according to any one of the preceding claims wherein the hydrogel comprises 2% by weight of chitosan and 1% by weight of water soluble anionic cyclodextrin polymer relative to the total weight of the hydrogel.

7. The hydrogel composition for use according to any one of the preceding claims wherein the hydrogel comprises 2% by weight of chitosan, 0.5% by weight of water soluble anionic cyclodextrin polymer and 0.5% by weight of water insoluble anionic cyclodextrin polymer relative to the total weight of the hydrogel.

8. The hydrogel composition for use according to any one of the preceding claims wherein the hydrogel composition comprises from 0.1 to 5%, preferably from 0.1 to 1%, by weight of a pharmacological agent relative to the total weight of the hydrogel composition.

9. A method for preparing a hydrogel composition as recited in any one of the preceding claims comprising a pharmacological agent, said method comprising the following steps:
(i) providing powders of water soluble anionic cyclodextrin polymer, water insoluble anionic cyclodextrin polymer powder when applicable and chitosan;
(ii) adding by wet granulation a pharmacological agent to the water soluble anionic cyclodextrin polymer powder or to the mixture of water soluble and water insoluble anionic cyclodextrin polymer powders;
(iii) dry mixing the powder obtained in step (ii) with chitosan powder in order to form a mixture of powders;
(iv) optionally sieving, or co-milling and sieving, the mixture of powders obtained in step (iii);
(v) suspending the mixture of powders obtained in step (iv) in an aqueous medium having a pH that enables the anionic cyclodextrin polymer(s) to be dissolved without dissolving the chitosan;
(vi) acidifying the suspension obtained from step (v) in order to form the hydrogel composition.

10. The method according to claim 9 wherein step (vi) is performed by addition of an acid selected from the group consisting of acetic acid, formic acid, tartaric acid, salicylic acid, glutamic acid, propanoic acid, hydrochloric acid, citric acid, lactic acid and mixtures thereof, preferably the acid is lactic acid or hydrochloric acid.

## Patentansprüche

1. Hydrogelzusammensetzung, umfassend:
(a) ein Hydrogel aus einem Polymernetzwerk, das Folgendes umfasst:
(i) Chitosan;
(ii) anionische Cyclodextrinpolymere, wobei es sich bei den anionischen Cyclodextrinpolymeren um wasserlösliche anionische Cyclodextrinpolymere oder um eine Mischung aus wasserlöslichen anionischen Cyclodextrinpolymeren und wasserunlöslichen anionischen Cyclodextrinpolymeren handelt (CDPi), wobei das Gewichtsverhältnis von wasserlöslichen anionischen Cyclodextrinpolymeren zu wasserunlöslichen anionischen Cyclodextrinpolymeren etwa 1:1 beträgt;
wobei das Gewichtsverhältnis von Chitosan zum Gesamtgewicht der anionischen Cyclodextrinpolymere etwa 2:1 beträgt, wobei der Begriff "etwa" bedeutet, dass der betreffende Wert um 10 %, insbesondere um 5 %, im Einzelnen um 1 % niedriger oder höher als der angegebene Wert ist; und
(b) gegebenenfalls einen in das Polymernetzwerk inkorporierten pharmakologischen Wirkstoff,
zur Verwendung bei der Behandlung von Gelenkerkrankungen.

2. Die Hydrogelzusammensetzung zur Verwendung gemäß Anspruch 1, wobei der pharmakologische Wirkstoff vorhanden ist und ein Analgetikum darstellt.

3. Die Hydrogelzusammensetzung zur Verwendung gemäß Anspruch 2, wobei der Analgetikum aus der Gruppe ausgewählt ist, die aus opioidhaltigen Analgetika, wie Codein, Oxycodon und Hydromorphon, und nichtsteroidalen Entzündungshemmern, wie Naproxen, Ibuprofen, Diflunisal und Ketorolac, besteht.

4. Die Hydrogelzusammensetzung zur Verwendung gemäß einem der vorstehenden Ansprüche, wobei es sich bei den Gelenkerkrankungen um Osteoarthritis, Osteoarthrose, rheumatische Erkrankungen und degenerative Meniskusläsionen handelt.

5. Die Hydrogelzusammensetzung zur Verwendung gemäß einem der vorstehenden Ansprüche, wobei die Hydrogelzusammensetzung durch intraartikuläre Injektion verabreicht wird.

6. Hydrogelzusammensetzung zur Verwendung gemäß einem der vorstehenden Ansprüche, wobei das Hydrogel 2 Gew.-% Chitosan und 1 Gew.-% wasserlösliches anionisches Cyclodextrinpolymer, bezogen auf das Gesamtgewicht des Hydrogels, umfasst.

7. Die Hydrogelzusammensetzung zur Verwendung gemäß einem der vorstehenden Ansprüche, wobei das Hydrogel 2 Gew.-% Chitosan, 0,5 Gew.-% wasserlösliches anionisches Cyclodextrinpolymer und 0,5 Gew.-% wasserunlösliches anionisches Cyclodextrinpolymer, bezogen auf das Gesamtgewicht des Hydrogels, umfasst.

8. Die Hydrogelzusammensetzung zur Verwendung gemäß einem der vorstehenden Ansprüche, wobei die Hydrogelzusammensetzung 0,1 bis 5 Gew.-%, vorzugsweise 0,1 bis 1 Gew.-%, eines pharmakologischen Wirkstoffs, bezogen auf das Gesamtgewicht der Hydrogelzusammensetzung, umfasst.

9. Verfahren zur Herstellung einer Hydrogelzusammensetzung gemäß einem der vorstehenden Ansprüche, die einen pharmakologischen Wirkstoff enthält, wobei das Verfahren die folgenden Schritte umfasst:
(i) Bereitstellen von Pulvern aus wasserlöslichem anionischem Cyclodextrinpolymer, gegebenenfalls wasserunlöslichem anionischem Cyclodextrinpolymerpulver und Chitosan;
(ii) Hinzufügen eines pharmakologischen Wirkstoffs durch Nassgranulierung zu dem wasserlöslichen anionischen Cyclodextrinpolymerpulver oder zu der Mischung aus wasserlöslichen und wasserunlöslichen anionischen Cyclodextrinpolymerpulvern;
(iii) Trockenmischen des in Schritt (ii) erhaltenen Pulvers mit Chitosanpulver, um eine Pulvermischung zu bilden;
(iv) gegebenenfalls Sieben oder gemeinsames Mahlen und Sieben der in Schritt (iii) erhaltenen Pulvermischung;
(v) Suspendieren der in Schritt (iv) erhaltenen Pulvermischung in einem wässrigen Medium mit einem pH-Wert, der es ermöglicht, das/die anionische(n) Cyclodextrinpolymer(e) aufzulösen, ohne das Chitosan aufzulösen;
(vi) Ansäuern der in Schritt (v) erhaltenen Suspension, um die Hydrogelzusammensetzung zu bilden.

10. Verfahren nach Anspruch 9, wobei Schritt (vi) durch Zugabe einer Säure durchgeführt wird, die aus der Gruppe bestehend aus Essigsäure, Ameisensäure, Weinsäure, Salicylsäure, Glutaminsäure, Propansäure, Salzsäure, Zitronensäure, Milchsäure und Mischungen davon, wobei die Säure vorzugsweise Milchsäure oder Salzsäure, ausgewählt ist.

## Revendications

1. Composition d'hydrogel comprenant :
(a) un hydrogel comportant un réseau polymérique comprenant :
(i) du chitosane ;
(ii) des polymères de cyclodextrine anioniques, lesdits polymères de cyclodextrine anioniques étant des polymères de cyclodextrine anioniques hydrosolubles ou un mélange de polymères de cyclodextrine anioniques hydrosolubles et de polymères de cyclodextrine anioniques insolubles dans l'eau (CDPi) dans lequel le rapport entre la masse des polymères de cyclodextrine anioniques hydrosolubles et la masse des polymères de cyclodextrine anioniques insolubles dans l'eau est d'environ 1:1 ;
dans laquelle le rapport entre la masse du chitosane et la masse totale des polymères de cyclodextrine anioniques est d'environ 2:1, le terme « environ » signifiant que la valeur concernée est inférieure ou supérieure de 10 %, notamment de 5 %, en particulier de 1 %, à la valeur indiquée ; et
(b) optionnellement un agent pharmacologique incorporé dans le réseau polymérique,
pour utilisation pour le traitement des troubles articulaires.

2. Composition d'hydrogel pour utilisation selon la revendication 1, dans laquelle l'agent pharmacologique est présent et est un agent analgésique.

3. Composition d'hydrogel pour utilisation selon la revendication 2, dans laquelle l'agent analgésique est sélectionné dans le groupe constitué d'agents analgésiques opioïdes, tels que la codéine, l'oxycodone et l'hydromorphone, et d'agents anti-inflammatoires non stéroïdiens, tels que le naproxène, l'ibuprofène, le diflunisal et le kétorolac.

4. Composition d'hydrogel pour utilisation selon l'une quelconque des revendications précédentes, dans laquelle les troubles articulaires sont l'arthrose, l'ostéoarthrose, les troubles rhumatismaux et les lésions méniscales dégénératives.

5. Composition d'hydrogel pour utilisation selon l'une quelconque des revendications précédentes, dans laquelle la composition d'hydrogel est administrée par injection intra-articulaire.

6. Composition d'hydrogel pour utilisation selon l'une quelconque des revendications précédentes, dans laquelle l'hydrogel comprend 2 % en poids de chitosane et 1 % en poids de polymère de cyclodextrine anionique soluble dans l'eau, par rapport au poids total de l'hydrogel.

7. Composition d'hydrogel pour utilisation selon l'une quelconque des revendications précédentes, dans laquelle l'hydrogel comprend 2 % en poids de chitosane, 0,5 % en poids de polymère de cyclodextrine anionique soluble dans l'eau et 0,5 % en poids de polymère de cyclodextrine anionique insoluble dans l'eau, par rapport au poids total de l'hydrogel.

8. Composition d'hydrogel pour utilisation selon l'une quelconque des revendications précédentes, dans laquelle la composition d'hydrogel comprend de 0,1 à 5 %, de préférence de 0,1 à 1 %, en poids d'un agent pharmacologique par rapport au poids total de la composition d'hydrogel.

9. Procédé de préparation d'une composition d'hydrogel telle que définie dans l'une quelconque des revendications précédentes, comprenant un agent pharmacologique, ledit procédé comprenant les étapes suivantes :
(i) fournir des poudres de polymère de cyclodextrine anionique hydrosoluble, de polymère de cyclodextrine anionique insoluble dans l'eau le cas échéant, et de chitosane ;
(ii) ajouter, par granulation humide, un agent pharmacologique à la poudre de polymère de cyclodextrine anionique soluble dans l'eau ou au mélange de poudres de polymères de cyclodextrine anioniques solubles et insolubles dans l'eau ;
(iii) mélanger à sec la poudre obtenue à l'étape (ii) avec de la poudre de chitosane afin de former un mélange de poudres ;
(iv) optionnellement tamiser, ou broyer conjointement puis tamiser, le mélange de poudres obtenu à l'étape (iii) ;
(v) mettre en suspension le mélange de poudres obtenu à l'étape (iv) dans un milieu aqueux ayant un pH permettant de dissoudre le ou les polymères de cyclodextrine anioniques sans dissoudre le chitosane ;
(vi) acidifier la suspension obtenue à l'étape (v) afin de former la composition d'hydrogel.

10. Procédé selon la revendication 9, dans lequel l'étape (vi) est réalisée par addition d'un acide sélectionné dans le groupe constitué de l'acide acétique, l'acide formique, l'acide tartrique, l'acide salicylique, l'acide glutamique, l'acide propanoïque, l'acide chlorhydrique, l'acide citrique, l'acide lactique et leurs mélanges, l'acide étant de préférence l'acide lactique ou l'acide chlorhydrique.
